# NEUE EUROPÄISCHE PATENTSCHRIFT

(11) **EP 0 371 909 B2**
(45) Veröffentlichungstag und Bekanntmachung des Hinweises auf die Entscheidung über den Einspruch: **07.03.2001**
(45) Hinweis auf die Patenterteilung: 15.09.1993
(21) Anmeldenummer: 89730212.1
(22) Anmeldetag: 18.11.1989
(51) Int. Cl.: A61B 19/08, A61B 19/02

(54) **Folienüberzug zum Schutz eines chirurgischen Instruments**
Sheath for the protection of a surgical instrument
Enveloppe de protection pour instrument chirurgical

(30) Priorität: 01.12.1988 DE 8815076 U; 20.04.1989 DE 8905102 U
(43) Veröffentlichungstag der Anmeldung: 06.06.1990
(73) Patentinhaber: BIOMET MERCK Deutschland GmbH, 12247 Berlin (DE)
(72) Erfinder: Anapliotis, Emmanuel, D-1000 Berlin 33 (DE)
(74) Vertreter: Gross, Felix, Dr.

(56) Entgegenhaltungen:
- EP-A- 0 210 257
- EP-A- 0 298 685
- WO-A-81/03609
- DE-A- 2 527 695
- DE-A- 3 136 802
- DE-A- 7 925 649
- DE-A- 8 815 549
- DE-C- 406 507
- DE-C- 808 362
- DE-U- 8 111 273
- DE-U- 8 812 027
- DE-U- 8 815 549
- GB-A- 1 541 390
- US-A- 3 809 072
- US-A- 3 866 601

## Beschreibung

Die Erfindung betrifft einen Folienüberzug der im Oberbegriff des Anspruchs 1 angegebenen Art.

Folienüberzüge sind gebräuchliche Hilfsmittel zum Schutz chirurgischer Instrumente vor septischen Verunreinigungen während der Operation. Insbesondere hochempfindliche optische Instrumente, wie beispielsweise Endoskope, Arthroskope oder chirurgische Laser erfordern schützende Umhüllungen, da ihre Sterilisation schwierig ist und sie außerdem nicht verunreinigt werden dürfen. Durch die Umhüllung mit einem sterilen schlauchartigen Folienüberzug jedoch erübrigt sich die Sterilisation der abgedeckten Teile. Folienüberzüge sind zum einmaligen Gebrauch bestimmt.

Aufgrund der Vielfalt der möglichen Anwendungen sind verschiedene in ihrer Form entsprechend dem Anwendungsfall variierende Folienüberzüge bekannt geworden. Vorwiegend weisen Folienüberzüge eine ausgeprägte Schlauchform auf mit Schlauchweiten im Zentimeter- bis Dezimeter-Bereich und Schlauchlängen im Dezimeter- bis Meter-Bereich. Bei großen Längen ist das das Überstreifen insbesondere enger Folienüberzüge bzw. das Einführen des entsprechenden chirurgischen Instrumentes häufig schwierig und erfordert einiges Geschick.

Ein schlauchförmiger Folienüberzug zum Schutz eines stabartigen chirurgischen Instruments ist aus der US-A- 3 809 072 bekannt. Dieser Folienschlauch dient zur sterilen Umhüllung einer Lichtsonde, die zur Beleuchtung einer Körperhöhle dient und weist an seinem einen Ende eine Einstecköffnung auf, an der Teile des Folienschlauchs zu Laschen thermoplastisch verformt sind, mittels derer die Hülle über die Lichtsonde gezogen werden kann.

Ein nach innen gefalteter, einseitig offener, schlauchförmiger Auffangbeutel für Körperflüssigkeiten ist aus der DE-C-0 406 507 bekannt. Mit einer derartigen Faltung kann die Gesamtlänge des Auffangbeutels wesentlich verringert werden, so daß der Platzbedarf geringer ist. Das Verfahren zur Erzeugung der Faltung ist aber nur für einseitig geschlossene Schläuche geeignet.

Aus dem nachveröffentlichten Dokument DE-A-8 815 549 ist eine steckgefaltete Schlauchfolie für Arthroskopiekameras bekannt. Auskleidungen des Öffnungsbereiches der Folie sind darin nicht beschrieben.

Außerdem ist ein Folienüberzug zum Schutz eines stabartigen chirurgischen Instrumentes mit einem eine Einstecköffnung aufweisenden Folienschlauch vorbekannt. Die Einstecköffnung besitzt zwei, einander gegenüberliegende, im wesentlichen deckungsgleiche Seitenkanten aufweisende, flexible, blattförmige Auskleidungen. Der Folienschlauch weist eine Steckfaltung und eine von der Einstecköffnung gegenüberliegende Ausgangsöffnung auf, die durch Abtrennung der Spitze entlang einer zu diesem Zweck vorgesehenen Perforation gebildet wird und durch die mindestens ein Teilbereich des stabartigen chirurgischen Instruments hindurchsteckbar ist.

Der Erfindung liegt die Aufgabe zugrunde, bei einem Folienüberzug der eingangs genannten Gattung die Handhabung, insbesondere hinsichtlich des Überstreifens des Überzuges bzw. des Einführens eines chirurgischen Instrumentes in den Folienüberzug zu erleichtern.

Darüberhinaus besteht die Aufgabe, ein einfaches Verfahren zur Steckfaltung des erfindungsgemäßen Folienüberzugs anzugeben.

Diese Aufgaben werden mit den kennzeichnenden Merkmalen der Ansprüche 1 bzw. 10 gelöst.

Der Erfindung liegt die Erkenntnis zugrunde, daß ein unkompliziertes Öffnen und Schließen eines flexiblen schlauchartigen Behältnisses, vorzugsweise eines Folienschlauches, durch eine Steckfaltung des Folienschlauchs und durch Verstärkung seines Öffnungsbereiches mittels zweier, in Ruhestellung flächig aneinandergrenzender, blattförmiger flexibler Auskleidungen erreichbar ist, wobei durch leichten Finger- bzw. Daumendruck auf einander gegenüberliegende Kantenbereiche der Auskleidungen deren maulartiges Auseinanderspreizen bewirkbar ist. Damit kann auch unter Operationsbedingungen einfach und schnell einhändig eine zum sicheren Einführen des Instrumentes ausreichend große und stabile Öffnung erzielt werden.

Durch Auskleidung des Öffnungsbereiches des Folienüberzuges mit zwei deckungsgleichen flexiblen Streifen, deren doppelte Länge dem Umfang des Öffnungsbereiches entspricht und deren Breite mindestens einige Zentimeter beträgt, ist neben der Vereinfachung auch eine Beschleunigung des Einführvorganges erreichbar. Jegliches Suchen der "richtigen" Einstecköffnung entfällt, da durch Druck auf die Seiten der Streifen immer die Einstecköffnung und nicht eine von der teleskopartigen Steckfaltung herrührende Seitenfalte aufgespreizt wird.

Als flexible Materialien sind vorzugsweise Pappstreifen, aber auch Kunststoff- oder Metallfolien geeignet. Die Materialstärke ist dabei so zu wählen, daß einerseits das manuelle Aufspreizen, und damit das Biegen der Auskleidungen über einen ausreichend langen Zeitraum ohne große Kraftanstrengung möglich ist und andererseits die Flexibilität des Materials weitgehend erhalten bleibt und nach Beendigung des Einführvorganges ein Zurückschnappen der beiden Auskleidungen in ihre Ausgangslage erfolgt.

Entsprechend einer vorteilhaften Weiterbildung der Erfindung wird das Aufspreizen der Auskleidungsteile durch die Verwendung von teilrohrförmige Vorzugswölbungen aufweisenden Auskleidungsteilen erleichtert, wobei diese verarbeitungs- und materialbedingten Vorzugswölbungen konvex nach außen weisen und sich in ihrer axialen Richtung in Längsrichtung des Folienüberzuges erstrecken.

Die Auskleidungsteile sind bevorzugt rechtekkig geformt und sind der Breite des Folienschlauchs angepaßt. Sie setzen den Folienschlauch in Richtung der Öffnung fort. Insbesondere ragen die Auskleidungsteile laschenartig über den Folienrand hinaus und sind mit Grifflöchern versehen, wodurch das Auseinanderziehen der teleskopartigen Steckfaltung weiter vereinfacht wird. Die Auskleidungen können innerhalb der Einstecköffnung des schlauchartigen Folienüberzuges eingeklebt oder eingeschweißt werden.

Zum Gebrauch wird das Instrument nach dem Aufspreizen der Auskleidungsteile in den noch zusammengefalteten Überzug eingeschoben und das distale Ende des Instruments mittels dort vorgesehener Befestigungsmittel am entsprechenden Ende des Überzugs fixiert. Durch Voranschieben des Instruments unter Festhalten der Auskleidungsteile wird das Instrument in den Folienüberzug eingeschoben, der sich damit gerade um die notwendige Länge entfaltet.

Zur Halterung von Kabeln und Schläuchen, die durch den Folienüberzug hindurchragen, sind vorzugsweise an der Innenseite entsprechende klammerartige Klemmhalterungen vorgesehen. Diese bestehen bevorzugt aus einem thermoplastischen Kunststoff und werden mittels durch Ausparungen gesteckter Enden und deren Warmverformung vernietet.

Gemäß einer Weiterbildung der Erfindung ist zur Vermeidung eines "Hängenbleibens" des Instruments die Faltung so gewählt, daß in Einführungsrichtung des Instruments hinter der Auskleidung an der Innenseite des zusammengefalteten Folienüberzugs nur Faltkanten vorhanden sind, die zur Ausgangsöffnung weisen, so daß ein Verhaken des Instruments, wie es bei entgegengesetzt zur Einführungsrichtung gerichteten Falten auftreten könnte, sicher verhindert ist. Auf diese Weise ist trotz Längenvariabilität des Überzugs verhindert, daß die Spitze des hindurchzuschiebenden Instrumentes, beispielsweise die Kanüle eines Arthroskops, in den seitlichen Falten der Steckfaltung hängenbleibt.

Gemäß einer anderen vorteilhaften Weiterbildung wird ein Verfahren angegeben, nach dem eine derartige Faltung mit einer einfachen Vorrichtung herstellbar ist.

Entsprechend diesem Verfahren zur Herstellung der Steckfaltung des Folienüberzuges wird der Schlauch auf einen Dorn eng anliegend aufgezogen, wonach eine der Einstecköffnung gegenüberliegende Austrittsöffnung koaxial nach außen umgeschlagen wird, bis sich diese etwa im Bereich der Einstecköffnung befindet. Eine dadurch entstandene freie Öffnung, die wiederum der Einstecköffnung gegenüberliegt, wird anschließend ebenfalls "umgestülpt" und bis einige Zentimeter unterhalb der Austrittsöffnung geschoben. Dadurch entsteht eine weitere freie Öffnung. Diese und einige nachfolgende freie Öffnungen werden auf die gleiche Weise "umgestülpt", wodurch die Folienschlauchlänge jeweils etwa halbiert wird. Auf diese Weise läßt sich die Länge des Folienschlauches auf das gewünschte Maß verkürzen. Das "Umkrempeln" der freien Öffnungen ist durch die Verwendung des Domes ein einfacher und zeitsparender Vorgang. Damit die Austrittsöffnung der Einstecköffnung gegenüberliegt und der gefaltete Folienschlauch wieder auseinanderziehbar ist, wird die Austrittsöffnung abschließend "umgestülpt" und der Folienschlauch von dem Dorn herabgezogen.

Nach der verfahrensgemäßen Faltung kann die Austrittsöffnung gegebenenfalls teilweise verschweißt werden, um entsprechende Anschläge für ein einzuschiebendes chirurgisches Instrument zu erzeugen bzw. für Befestigszwecke mit Klebestreifen versehen werden.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1a einen Folienüberzug für Arthroskope in zusammengefalteter Form in Draufsicht mit schematischer Darstellug der Faltung,
Figur 1b die Seitenansicht zu Figur 1a,
Figur 2 den Folienüberzug gemäß Figur 1a mit manuell aufgespreizter Einstecköffnung,
Figur 3a die aufgespreizte Auskleidung der Einstecköffnung als Einzelteil gemäß Figur 2,
Figur 3b die Ausgangsposition der Auskleidung der Einstecköffnung gemäß den Figuren 1a und 1b,
Figur 4 den Folienüberzug gemäß Figur 1a während des Überstreifvorganges über ein Arthroskop,
Figur 5 den Öffnungsbereich eines Folienüberzugs mit einer eine Grifflasche aufweisenden Auskleidung,
Figur 6 eine Kabelhalterung innerhalb eines angedeuteten aufgespreizten Auskleidungsteiles, die
Figuren 7a bis 7d schematische Veranschauli-chungen der Verfahrensschritte einer Ausführungsvariante des erfindungsgemäßen Verfahrens zur Herstellung der Steckfaltung eines Folienschlauches und die
Figuren 8a bis 8c entsprechende Darstellungen zu Verfahrensschritten einer weiteren Ausführungsvariante des erfindungsgemäßen Verfahrens zur Herstellung der Steckfaltung eines Folienschlauches in schematisierter Darstellung.

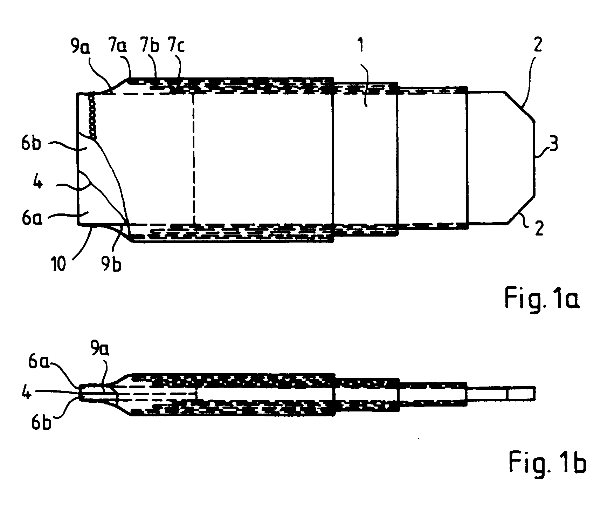

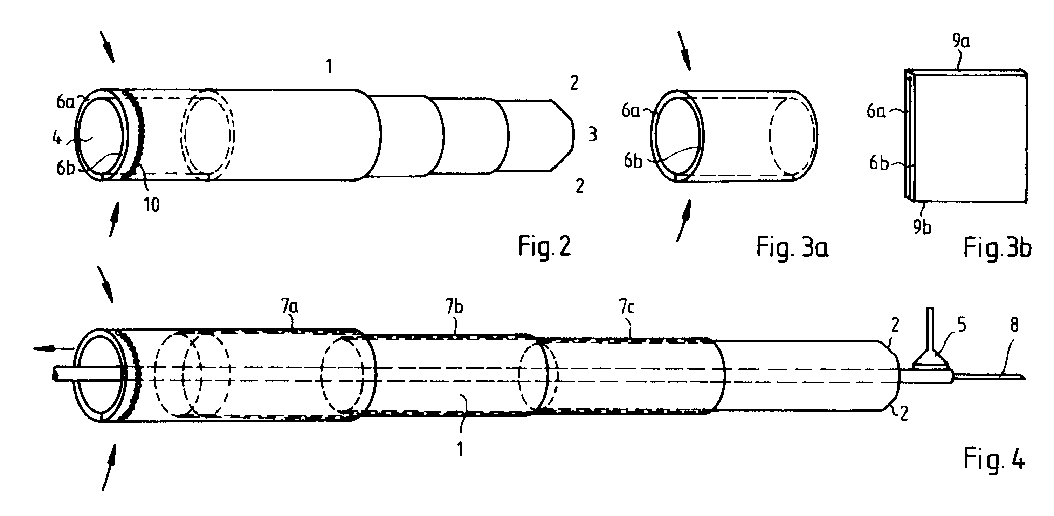

In der Verkaufskonfiguration ist der Folienüberzug, wie in den Figuren 1a und 1b dargestellt, zusammengefaltet. Ein Folienschlauch 1 ist in seiner Längsrichtung teleskopartig abschnittweise ineinandergeschoben, so daß seine Gesamtlänge wesentlich verkürzt erscheint. Die Art der Faltung ist am oberen Rand der Darstellung der Figur 1a schematisch und stark vergrößert wiedergegeben. Durch einen derartig verkürzten Schlauch 1 läßt sich ein Arthroskop, wie in Figur 4 dargestellt, leicht hindurchschieben. Zum Fixieren der gewünschten Einschiebtiefe des Arthroskopes in den Folienschlauch dienen sackartige Anschläge 2. Das Arthroskop läßt sich durch eine kleine Öffnung 3 an dem der Einstecköffnung 4 entgegengesetzten Ende des Folienüberzuges bis über die externen Anschlüsse 5 für die Saug- und Spülkanäle in den Folienschlauch 1 einschieben. Zur Erleichterung des Einschiebevorganges ist die Einstecköffnung 4 innenseitig mit zwei gleichgroßen federnd gegeneinander maulartig aufspreizbaren Auskleidungen 6a und 6b ausgestattet. Die Auskleidungen 6a und 6b liegen zunächst flach übereinander. Diese Ausgangsposition ist in den Figuren 1 a, 1b und 3b dargestellt. Die Auskleidungen 6a und 6b ragen soweit in die Einstecköffnung hinein, daß sie die Faltensäume 7a, 7b und 7c der Ausgangsfaltung des Folienschlauches 1 überragen. Auf diese Weise wird vermieden, daß sich die Kanüle 8 des Arthroskopes beim Einführen in den Folienüberzug in einer Seitenfalte verfängt. Zum Einführen des Arthroskopes wird durch leichten Druck auf die flachen Seiten 9a und 9b der mit den Auskleidungen 6a und 6b versehenen Einstecköffnung 4 ein maulförmiges Aufspreizen der Einstecköffnung 4 bewirkt. Der Aufspreizvorgang ist aus den Figuren 2, 3a und 4 ersichtlich, wobei die Kraftwirkungsrichtungen durch Pfeile dargestellt sind.

Die Auskleidungen 6a und 6b sind vorzugsweise aus Karton hergestellt. Wie in den Figuren 3a und 3b erkennbar, genügt dazu ein einziges rechteckiges Stück Karton, das in der Mitte gefaltet wird, so daß zwei gleichgroße übereinanderliegende Teile entstehen. Das Befestigen des gefalteten Kartons innerhalb der Einstecköffnung 4 des Folienüberzuges läßt sich mittels einer Schweißnaht 10 problemlos und schnell realisieren.

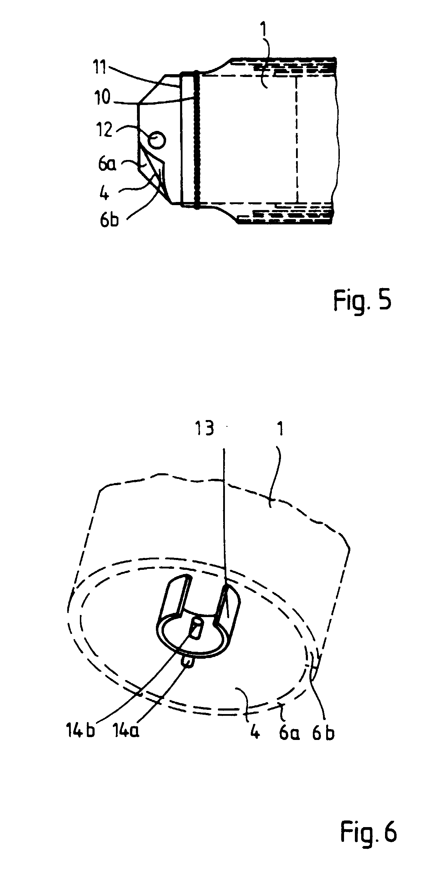

In Figur 5 ist eine weitere Ausführung des Einsteckbereiches eines Folienüberzuges in Draufsicht dargestellt. Die Auskleidungen 6a und 6b ragen über den Folienrand 11 hinaus und sind mit jeweils einem Griffloch 12 versehen. Auf diese Weise wird das Auseinanderziehen des Folienüberzuges noch weiter vereinfacht. Außerdem läßt sich der unsterile Bereich auf die über den Rand 11 des Folienschlauches 1 hinausragende Lasche mit dem Griffloch 12 begrenzen.

Figur 6 zeigt eine Halterung für ein Kabel oder einen Zuführungsschlauch, welche innerhalb einer gestrichelt angedeuteten Einstecköffnung 4 angeordnet ist. Die Halterung besteht aus einer schellenartigen elastisch aufspreizbaren Kabel- oder Schlauchumklammerung 13 und zwei Befestigungsnieten 14a und 14b, welche mit dem Foilenschlauch 1 im Bereich der Einstecköffnung 4 durch Heißvernieten des thermoplastischen Werkstoffs befestigt sind.

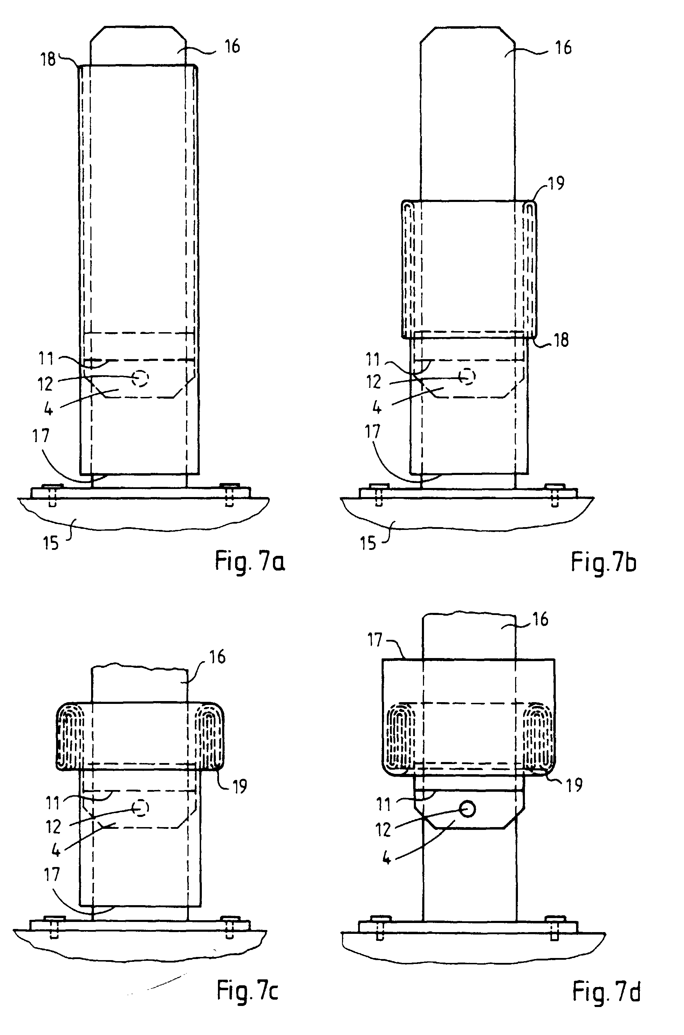

Die Figuren 7a bis 7d veranschaulichen in schematischer Darstellung eine Ausführungsvariante des erfindungsgemäßen Verfahrens zur Herstellung der Steckfaltung eines Folienschlauches 1. Der Folienschlauch 1 wird mit seiner Einstecköffnung 4 über einen mit einer festen Unterlage 15 verschraubten rohr- oder zylinderförmigen Dorn 16 gezogen. Der Durchmesser des Dorns 16 entspricht dem Innendurchmesser des Folienschlauches 1.

Nach einem in Figur 7a dargestellten Verfahrensschritt wird die der Einstecköffnung gegenüberliegende Ausgangsöffnung 17 nach außen umgewendet und über den auf dem Dorn 16 fest aufgezogenen Folienschlauch 1 hinweg bis unterhalb der Einstecköffnung 4 geschoben.

Figur 7b zeigt, wie eine dadurch entstandene erste freie Öffnung 18 nach außen "umgekrempelt" und bis wenig unterhalb der Einstecköffnung 4 geschoben wird. Dieser Vorgang wird noch einmal - wie Figur 7c zeigt - mit einer zweiten durch den "Umkrempelprozeß" entstandenen freien Öffnung 19 wiederholt.

Abschließend wird die überstehende Ausgangsöffnung 17 noch einmal nach außen umgewendet und gemäß Figur 7d umgewendet, wobei sie die Faltenbereiche des Folienschlauches 1 überragt. Der auf diese Weise gefaltete Folienschlauch 1 wird vom Dorn 16 heruntergezogen und die Ausgangsöffnung 17 kann teilweise zugeschweißt oder mit Klebestreifen versehen werden. Dadurch erhält der zusammengefaltete Folienschlauch seine konfektionierte zur Benutzung bereite Form bei der die Einführungsöffnung zum Aufspreizen und Einführen des Instruments ergriffen werden kann. Auch die Endöffnung ist erreichbar, damit das Ende des Instruments mit dieser verbunden werden kann, so daß sich der Überzug beim weiteren Voranschieben des Instruments entfaltet.

Bei den bisher dargestellten Verfahrenschritten wird in vorteilhafter Weise ausgenutzt, daß der Folienschlauch mit seiner Innenseite an dem zylindrischen Dorn anhaftet und so nach dem Umstülpen an dem umgestülpten Ende leicht heruntergezogen werden kann, wobei die Folienbereiche untereinander gleiten.

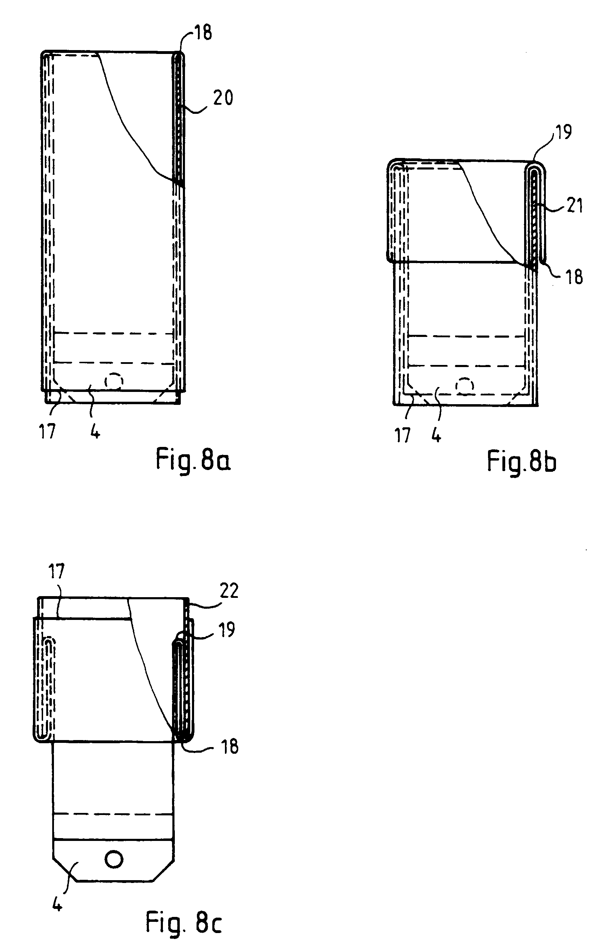

In den Figuren 8a bis 8c sind die Verfahrensschritte eines weiteren Ausführungsbeispiels des erfindungsgemäßen Verfahrens zur Herstellung der Steckfaltung schematisch wiedergegeben, wobei die dargestellten den Umstülpvorgang unterstützenden Rohre auch bei weiteren Schritten des vorstehend beschriebenen Verfahrens wahlweise verwendet werden können.

Statt des Domes 16 der vorstehend beschriebenen Variante kommt hier ein Satz verschieden langer dünnwandiger Rohre zum Einsatz. Der Folienschlauch 1 wird - wie Figur 8a zeigt - zunächst mit dem die Einstecköffnung 4 aufweisenden Ende in ein erstes Rohr 20 eingeschoben. Die Länge des Rohres 20 entspricht ungefähr der halben Schlauchlänge, so daß das über den Rand des Rohres 20 hinausragende Ende mit der Ausgangsöffnung 17 nach außen umgewendet und über das Rohr geschoben werden kann bis sich die Ausgangsöffnung 17 etwa in Höhe der Einstecköffnung 4 befindet. Anschließend wird ein zweites kürzeres Rohr 21 soweit über das erste Rohr 20 und den umgewendeten Teil des Folienschlauches 1 aufgeschoben, bis das erste über das zweite Rohr hinausragt. Um den die erste freie Öffnung 18 bildenden Wendeknick nach außen über das zweite Rohr 21 hinweg umwenden zu können, wird das lange erste Rohr 20 durch Abziehen entfernt. Das Umwenden wird mit immer kürzer werdenden Rohren sinngemäß mehrmals wiederholt. Ein letztes Rohr 22 wird nur soweit aufgeschoben, bis sich dessen unterer Rand oberhalb der Ausgangsöffnung 17 befindet. Das dem vorangegangenen Verfahrensschritt zugeordnete Rohr wird entfernt und die Ausgangsöffnung 17 läßt sich nach außen umwenden, wodurch diese in zur Einstecköffnung 4 entgegengesetzte Richtung weist. Der gefaltete und von dem letzten Rohr 22 abgezogene Folienschlauch 1 steht damit einer weiteren Bearbeitung zur Verfügung.

Nach vorbeschriebenen Verfahren wird eine Faltung erreicht, bei der - wegen des fortwährenden Umschlagens des überzustülpenden Endes des (mehrlagigen) Schlauchs nach außen - keine das Einschieben des chirurgischen Instruments behindernden Faltenkanten an der Innenseite des noch zusammengefalteten Überzugs entstehen. Die Innenwandung weist in Einführungsrichtung keinerlei Hindernisse auf.

Bei dem zylinderförmigen Dorn bzw. Rohr kann es sich auch um ein entsprechendes Profil anderer Querschnittsform handeln. Insbesondere einen Flachkörper anstelle des Dorns oder auch ein nicht ringförmig geschlossenes Profil anstelle des Rohrs können vorteilhaft verwendet werden.

## Patentansprüche

1. Folienüberzug zum Schutz eines stabartigen chirurgischen Instruments mit einem eine Einstecköffnung (4) aufweisenden Folienschlauch (1), wobei die Einstecköffnung (4) zwei, einander gegenüberliegende, im wesentlichen deckungsgleiche Seitenkanten (9a, 9b) aufweisende, flexible, blattförmige Auskleidungen (6a/6b) aufweist, wobei der Folienschlauch (1) eine Steckfaltung und eine von der Einstecköffnung (4) gegenüberliegende Ausgangsöffnung (17) aufweist, durch die mindestens ein Teilbereich des stabartigen chirurgischen Instruments hindurchsteckbar ist,
dadurch gekennzeichnet,
daß die den Folienschlauch (1) im Bereich der Einstecköffnung (4) verstärkenden Auskleidungen (6a/6b) bei geschlossener Einstecköffnung (4) vollflächig aufeinanderliegen und soweit in die Einstecköffnung (4) hineinragen, daß sie die zu der Einstecköffnung hinweisenden Faltensäume (7a, 7b, 7c) der Steckfaltung überragen, und daß zur Öffnung der Einstecköffnung (4) die Auskleidungen (6a/6b) durch Druck auf die Seitenkanten (9a, 9b) maulartig auseinanderspreizbar sind.

2. Folienüberzug nach Anspruch 1 , **dadurch gekennzeichnet**, daß die Auskleidungen (6a/6b) aus entsprechend steifem Karton oder Kunststoff- bzw. Metallfolie bestehen.

3. Folienüberzug nach einem der vorangehenden Ansprüche, **dadurch gekennzeichnet**, daß die Auskleidungen (6a/6b) im wesentlichen Rechteckform aufweisen.

4. Folienüberzug nach einem der vorangehenden Ansprüche, **dadurch gekennzeichnet**, daß die Auskleidungen (6a/6b) über den Folienrand (11) hinausragen und insbesondere mit Grifflöchern oder -aussparungen (12) versehen sind.

5. Folienüberzug nach einem der vorangehenden Ansprüche, **dadurch gekennzeichnet**, daß die Auskleidungen (6a/6b) an der Innenseite der Einstecköffnung (4) mit der Folie des Überzugs verklebt oder verschweißt sind.

6. Folienüberzug nach einem der vorangehenden Ansprüche, **dadurch gekennzeichnet**, daß die Auskleidungen (6a/6b) durch mittiges Falten aus einem rechteckigen Flächenteil gebildet sind, wobei eine Faltkante an einer inneren Begrenzung der Einstecköffnung (4) anliegt und die Breite der Auskleidungen (6a/6b) jeweils dem halben Umfang der Einstecköffnung (4) entspricht.

7. Folienüberzug nach einem der vorangehenden Ansprüche, **dadurch gekennzeichnet**, daß die Auskleidungen (6a/6b) nach außen weisende, teilrohrförmige Vorzugswölbungen aufweisen, deren Achse sich in Richtung der Längsausdehnung des Folienschlauchs (1) erstrekken, wobei die Vorzugswölbung insbesondere bei Kartonwerkstoffen der aufgrund der Herstellung entstandenen Vorzugswölbung entspricht.

8. Folienüberzug nach einem der vorangehenden Ansprüche, **dadurch gekennzeichnet**, daß mindestens eine klammerartige Kabel- und/oder Schlauchhalterung vorgesehen ist, die insbesondere einen elastisch verformbaren U-förmigen Klemmbereich (13) aufweist, wobei die Kabelhalterung innerhalb der Einstecköffnung (4) mit dieser verbunden, insbesondere durch Warmverformung vernietet, ist.

9. Folienüberzug nach einem der vorangehenden Ansprüche, **dadurch gekennzeichnet**, daß die Steckfaltung des Folienschlauchs (1) teleskopartig ist.

10. Verfahren zur Herstellung der teleskopartigen Steckfaltung des Folienschlauchs (1) des Folienüberzuges nach Anspruch 9,
**gekennzeichnet durch**
folgende Verfahrensschritte:
a. Aufziehen des Folienschlauchs (1) auf einen, insbesondere zylinderförmigen, Dorn (16) oder Einlegen des Folienschlauchs (1) in ein Rohr (20) bzw. einen entsprechenden Körper mit dem die Einstecköffnung (4) aufweisenden Ende voran wobei der Außendurchmesser von Dorn (16) oder Rohr (20) dem Innendurchmesser des Folienschlauchs (1) und deren Länge mindestens der halben Länge des Folienschlauchs (1) entspricht,
b. wenden des Endes mit der der Einstecköffnung (4) gegenüberliegenden Ausgangsöffnung (17) unter Umstülpen nach außen sowie koaxiales Verschieben der Ausgangsöffnung (17) bis diese sich etwa im Bereich der Einstecköffnung (4) befindet, insbesondere nach Aufschieben eines weiteren Rohres (21) mit erweitertem Durchmesser über das erste Rohr (20) oder den Dorn (16) und Umstülpen über dieses Rohr (21), sowie
c. Wenden der der Einstecköffnung (4) gegenüberliegenden neu gebildeten freien Öffnung (18, 19), Umstülpen nach außen sowie koaxiales Verschieben der freien Öffnung (18, 19) bis diese sich etwa im Bereich der Einstecköffnung (4) befindet, insbesondere nach Aufschieben eines weiteren Rohres (22) mit erweitertem Durchmesser über das erste Rohr (20) oder den Dorn (16) und Umstülpen über dieses Rohr (22), sowie
d. gegebenenfalls mehrmaliges sinngemäßes wiederholen des vorangegangenen Verfahrensschrittes.

11. Verfahren nach Anspruch 10, **gekennzeichnet durch** den weiteren Verfahrensschritt:
e. Wenden der Ausgangsöffnung (17) nach außen, Umstülpen und koaxiales Verschieben der Ausgangsöffnung (17) gegebenenfalls nach Aufschieben eines weiteren Rohres (22) und Umstülpen über dieses Rohr (22).

12. Verfahren nach Anspruch 11, **gekennzeichnet durch** den weiteren Verfahrensschritt des Verschiebens aller der ersten freien Öffnung (18) nachfolgenden freien Öffnungen (19) soweit, bis diese mit der ersten freien Öffnung (18) abschließen.

## Claims

1. A sheeting cover for the protection of a rod-like surgical instrument, having tubular sheeting (1) with an insertion opening (4), this insertion opening (4) having two flexible lamellar linings (6a/6b) which have opposite, substantially congruent edges (9a,9b), wherein the sheeting cover (1) has a retractable fold and an exit opening (17) positioned opposite the insertion opening (4), through which at least a partial section of the rod-like surgical instrument may be inserted,
characterised in that when the insertion opening (4) is closed, the linings (6a/6b) reinforcing the tubular sheeting (1) in the area of the insertion opening (4) lie on top of one another over their entire surface, and project into the insertion opening (4) so far that they reach
the tucked hems (7a, 7b, 7c) of the retractable fold which point towards the insertion opening and that the linings (6a/6b) may be spread open like jaws by applying pressure to the side edges (9a,9b) in order to open the insertion opening (4).

2. A sheeting cover according to claim 1,
characterised in that the linings (6a/6b) are made of an appropriately stiff cardboard or plastics or metal film.

3. A sheeting cover according to any one of the preceding claims, characterised in that the linings (6a/6b) have a substantially rectangular form.

4. A sheeting cover according to any one of the preceding claims, characterised in that the linings (6a/6b) project beyond the rim (11) of the sheeting and are, in particular, provided with grip holes or recesses (12).

5. A sheeting cover according to any one of the preceding claims, characterised in that the linings (6a/6b) are glued to or welded to the sheeting of the cover on the inside of the insertion opening (4).

6. A sheeting cover according to any one of the preceding claims, characterised in that the linings (6a/6b) are formed by folding a rectangular surface section centrally, one folded edge bearing against an inner boundary of the insertion opening (4) and the widths of the linings (6a/6b) corresponding, in each case, to half the circumference of the insertion opening (4).

7. A sheeting cover according to any one of the preceding claims, characterised in that the linings (6a/6b) have outwardly oriented, tubular segment-like preferred curvatures, the axes of which extend in the direction of the longitudinal extent of the tubular sheeting (1), the preferred curvature in cardboard materials, in particular, corresponding to the preferred curvature obtained during manufacture.

8. A sheeting cover according to any one of the preceding claims, characterised in that a clamp-like cable and/or tube bracket is provided which has, in particular, a resiliently deformable U-shaped clamping region (13), the cable support being connected, inside the insertion port (4), to said opening and more particularly riveted thereto by hot-forming.

9. A sheeting cover according to any one of the preceding claims, characterised in that the retractable folds of the tubular sheeting (1) of the sheeting cover are telescopic.

10. A process for producing the telescopic retractable folds of the tubular sheeting (1) of the sheeting cover according to claim 9, characterised by the following steps:
a. Pushing the tubular sheeting (1) onto a pin (16), more particularly in the form of a cylinder, or inserting the tubular sheeting (1) into a tube (20) or a corresponding body, starting with the end providing the insertion port (4), the outer diameter of the pin (16) or tube (20) corresponding to the inner diameter of the tubular sheeting (1) and the length of said pin or tube corresponding to at least half the length of the tubular sheeting (1),
b. Turning over the end providing the exit port (17) positioned opposite the insertion opening (4), by turning the exit port (17) inside out as well as coaxially displacing it until it is more or less positioned in the region of the insertion opening (4), in particular after a further tube (21) with a larger diameter has been pushed onto the first tube (20) or the pin (16) and turning it over said tube (21) as well as
c. Turning over the newly formed free opening (18,19) positioned opposite the insertion port, turning the free opening (18,19) inside out as well as coaxially displacing it until it is positioned more or less within the region of the insertion opening (4), more particularly after a further tube (22) with a larger diameter has been pushed onto the first tube (20) or the pin (16) and turning it over said tube (22), as well as
d. Optionally repeating the preceding steps several times, in an analogous manner.

11. A process according to claim 10, characterised by the further step:
e. Turning the exit opening (17) outwards, turning the exit opening (17) inside out and coaxially displacing it, optionally after a further tube (22) has been pushed on and turning it over said tube (22).

12. A process according to claim 11, characterised by the further step of displacing all the free openings (19) following the first free opening (18) until said openings (19) end with the first free opening (18).

## Revendications

1. Housse faite d'un matériau en film ou en feuille pour protéger un instrument chirurgical semblable à une tige, comprenant une gaine (1) en film ou en feuille, possédant une ouverture d'introduction (4) qui présente deux garnissages (6a/6b) souples, en forme de feuilles ayant des bords latéraux (9a, 9b) qui se recouvrent essentiellement, où
la gaine (1) est pliée à emboîtement et est pourvue d'une ouverture de sortie (17) située à l'opposé de l'ouverture d'introduction (4) et à travers laquelle peut être enfilée au moins une partie de l'instrument chirurgical semblable à une tige, caractérisée en ce que les garnissages (6a/6b), renforçant la gaine (1) dans la zone de l'ouverture d'introduction (4), s'appliquent l'un contre l'autre par toute leur étendue lorsque l'ouverture d'introduction (4) est fermée et avancent sur une telle distance dans l'ouverture d'introduction qu'ils s'étendent au-delà des bords de plis (7a, 7b et 7c) de la configuration pliée de départ de la gaine 1 et que, pour produire l'ouverture de celle-ci, les garnissages (6a/6b) peuvent être écartés l'un de l'autre à la façon des lèvres d'une bouche par une pression sur les côtés latéraux (9a, 9b).

2. Housse selon la revendication 1, caractérisée en ce que les garnissages (6a/6b) sont réalisés d'un carton ayant la raideur requise ou d'une feuille de matière plastique ou de métal.

3. Housse selon une des revendications précédentes, caractérisée en ce que les garnissages (6a/6b) ont une forme essentiellement rectangulaire.

4. Housse selon une des revendications précédentes, caractérisée en ce que les garnissages (6a/6b) dépassent du bord (11) du film ou de la feuille et sont pourvus en particulier de trous ou d'évidements de préhension (12).

5. Housse selon une des revendications précédentes, caractérisée en ce que les garnissages (6a/6b) sont collés ou soudés au film ou à la feuille formant la housse, sur le côté intérieur de l'ouverture d'introduction (4).

6. Housse selon une des revendications précédentes, caractérisée en ce que les garnissages (6a/6b) sont formés d'un morceau rectangulaire de matériau plat qui est plié au milieu, le bord formé par le pli étant appliqué contre une délimitation intérieure de l'ouverture d'introduction (4) et la largeur de chacun des garnissages (6a/6b) correspondant à la moitié du périmètre de l'ouverture d'introduction (4).

7. Housse selon une des revendications précédentes, caractérisée en ce que les garnissages (6a/6b) présentent des bombements préférentiels dirigés vers l'extérieur et ayant la forme d'une partie de tube, dont l'axe est orienté suivant l'étendue longitudinale de la gaine (1), le bombement correspondant en particulier, s'il s'agit de matériaux de type carton, au bombement préférentiel créé lors de la fabrication.

8. Housse selon une des revendications précédentes, caractérisée en ce qu'elle comprend au moins un support de câble et/ou de tuyau souple, semblable à une pince, qui présente en particulier une partie de serrage (13) élastiquement déformable, de forme en U, le support de câble étant relié à l'ouverture d'introduction (4), à l'intérieur de celle-ci, notamment par rivetage avec déformation à chaud.

9. Housse selon une des revendications précédentes, caractérisée en ce que le pliage à emboîtement de la gaine (1) est de type télescopique.

10. Procédé pour réaliser le pliage à emboîtement télescopique de la gaine (1) de la housse selon la revendication (9), caractérisé par les étapes de procédé suivantes :
a. Enfilage de la gaine (1) sur un mandrin (16), notamment de forme cylindrique, ou disposition de la gaine (1) dans un tube (20) ou un corps analogue, avec l'extrémité présentant l'ouverture d'introduction (4) à l'avant, le diamètre extérieur du mandrin (16) ou du tube (20) correspondant au diamètre intérieur de la gaine (1) et sa longueur correspondant au moins à la moitié de la longueur de la gaine (1),
b. retournement de l'extrémité présentant l'ouverture de sortie (17), située à l'opposé de l'ouverture d'introduction (4), avec rabattement vers l'extérieur ainsi que déplacement coaxial de l'ouverture de sortie (17) jusqu'à ce que celle-ci soit située à peu près dans la zone de l'ouverture d'introduction (4), notamment après enfilage d'un tube (21) supplémentaire de plus grand diamètre par-dessus le premier tube (20) ou le mandrin (16) et rabattement par-dessus ce tube,
c. retournement de l'ouverture libre (18, 19) nouvellement formée et située à l'opposé de l'ouverture d'introduction (4), rabattement vers l'extérieur ainsi que déplacement coaxial de l'ouverture libre (18, 19) jusqu'à ce que celle-ci soit située à peu près dans la zone de l'ouverture d'introduction (4), en particulier après enfilage d'un tube supplémentaire (22) de plus grand diamètre par-dessus le premier tube (20) ou le mandrin (16), et rabattement par-dessus ce tube supplémentaire (22), ainsi que
d. répétition, de façon analogue, éventuellement à plusieurs reprises, de l'étape précédente du procédé.

11. Procédé selon la revendication 10, caractérisé par l'étape de procédé supplémentaire :
e. retournement de l'ouverture de sortie (17) vers l'extérieur, rabattement et déplacement coaxial de l'ouverture de sortie (17), éventuellement après enfilage d'un tube supplémentaire (22), et rabattement par-dessus ce tube supplémentaire (22).

12. Procédé selon la revendication 11, caractérisé par l'étape de procédé supplémentaire qui consiste à déplacer toutes les ouvertures libres (19) qui suivent la première ouverture libre (18), jusqu'à ce qu'elles soient alignées avec la première ouverture libre (18).
